# EUROPEAN PATENT SPECIFICATION

(11) **EP 3 052 061 B1**
(45) Date of publication and mention of the grant of the patent: **14.11.2018**
(21) Application number: 14796251.8
(22) Date of filing: 24.09.2014
(51) Int. Cl.: A61F 5/02

(54) **ORTHOPAEDIC CORSET**
ORTHOPÄDISCHES KORSETT
CORSET ORTHOPÉDIQUE

(30) Priority: 04.10.2013 IT AN20130183
(43) Date of publication of application: 10.08.2016
(73) Proprietor: Di Franco, Marco, 65121 Pescara (IT); Pallini, Domenico, 65121 Pescara (IT)
(72) Inventor: Di Franco, Marco, 65121 Pescara (IT); Pallini, Domenico, 65121 Pescara (IT)
(74) Representative: Premru, Rok
(86) International application number: PCT/IB2014/002062
(87) International publication number: WO 2015/049579

(56) References cited:
- EP-A1- 1 685 814
- EP-A2- 0 234 372
- DE-C- 55 101
- DE-C- 931 975
- FR-A- 328 326

## Description

The object of the present invention is an adjustable orthopaedic corset usable for treating temporary or chronic pathologies that afflict the spine.

The invention therefore falls within the field of medical products for treating pathologies affecting the spine.

More precisely, the invention relates to the field of orthoses used for the stabilisation and hyperextension of the spine following spine surgery and or in presence of pathologies such as, by way of a non-limiting example, oncologic or degenerative pathologies (tumours, metastasis, osteoporosis, etc.), adult dorsal scoliosis and hyperkyphosis, traumatic fractures of the thoracic-lumbar segment of the spine.

It is known that patients suffering from pathologies of traumatic, degenerative or inflammatory nature to the spine must use special orthoses, for example of the "corset" type, if they want to lead a life that is as natural, independent and qualitatively satisfying as possible or avoid relapses and pain.

Such orthoses have the task of receiving and supporting the trunk of the patient and absorbing the various stresses which develop on the spine, for example during walking.

At present, numerous and various types of orthopaedic corsets are know which perform the above mentioned role.

Among the most used there are those "spinal orthoses" consisting in a rigid frame capable of surrounding, like a "cage", the trunk of the patient between the pelvic girdle and the scapulo-humeral one and ensuring an adequate hyperextension of the spine.

The "spinal orthoses" most commonly available on the market provide, at the back, a rigid dorsal element configured to be locked against the back of the patient, and on the front an abdominal elastic band made of two fastenable halves (for example, through velcro).

Iliac shells, consisting in a curvilinear band which follows and closely surrounds the pelvic girdle of the patient, and vertical rods, ending with an axillary support which press against the sides of the patient, indissolubly connect the dorsal element to the abdominal elastic band.

Special adjusting means allow adapting the corset to the anatomical features of the patient's body
Such a spinal orthosis is therefore a single-block body which surrounds the body of the patient; this entails drawbacks which are well known to the man skilled in the art.

The monolithic structure of the spinal orthosis of the type just described and the relative overall dimensions make it particularly difficult to apply it in patients forced to immobility (for example laid in bed) by severe and painful pathologies. It is known that the orthopaedic technician (or any person in charge for the "dressing") must first place such corset behind the patient close to his\her back and then, after opening the two halves of the abdominal elastic band, apply a front pulling movement to bring the dorsal element and the vertical rods in contact, respectively, with the back and the sides of the torso.

Such operations may therefore be performed only if the patient is, or is put, in upright position.

The lifting of the body of the patient from a laid position to a substantially upright position, even if assigned to highly qualified and trained personnel, is liable to generate relapses and/or pain, that is greater depending on the severity of the pathology suffered from the patient or how recent the surgery undergone is.

The so-called "rigid three-point braces" (also know with the acronym C34 or C35 or C37), while representing a valid alternative, do not resolve optimally the drawbacks just mentioned and they add others.

The presence of a cumbersome pelvic band, for example, makes those braces C34/C35/C37 uncomfortable during the most usual movements, for example when sitting, as well as the sternal plate may result uncomfortable especially for those persons suffering from respiratory problems, very often associated to pathologies of the spine (e.g., in the case of hyperkyphosis, crush fractures or similar).

Such problems are found also for the orthopaedic corset described in EP 1 685 814 which is considered to represent the closest prior art, and which comprises, in fact, the uncomfortable sternal plate.

The orthopaedic corset described in the document DE 55 101, instead, does not seem particularly efficient for supporting the patient's torso and for absorbing the relative stresses; in other words, such corset seems an antiquated and outdated solution.

The object of the present invention is to eliminate the drawbacks of the prior art, by designing an orthopaedic corset for the support of the spine that can be easily and quickly worn and minimizing the movements of the patient.

A further object of the present invention is to provide an orthopaedic corset which makes advantageous for the orthopaedic technician or the person in charge for the dressing both the application and any subsequent adjustments, minimizing the postoperative stay of the patients and the subsequent rehabilitation.

A further object of the present invention is to provide an adjustable orthopaedic corset for the support of the spine which does not cause inconveniences to the patient wearing it.

These and other objects are achieved by the invention, with the features listed in the annexed independent claim 1.

Further features of the present invention shall be better highlighted by the following description of a preferred embodiment, in accordance with the patent claims and illustrated in the enclosed designs. Such figures should be considered as having an illustrative and non-limiting purpose, in which:
- Fig. 1 is a schematic view of the orthopaedic corset of the invention broken down in its front and dorsal part;
- Fig. 2 is an exploded view of the front part of the orthopaedic corset of Fig. 1;
- Fig. 3 is an exploded view of the dorsal part of the orthopaedic corset of Fig. 1;

The features of the invention shall now be described, using the reference numerals contained in the annexed figures. It should be noted that any dimensional and spatial term (such as "lower", "upper", "right", "left" and the like) refers, unless otherwise indicated, to the position according to which the object of the invention is shown in the drawings.

With the purpose of highlighting some features instead of others, non necessarily what described in the annexed drawings is perfectly scaled.

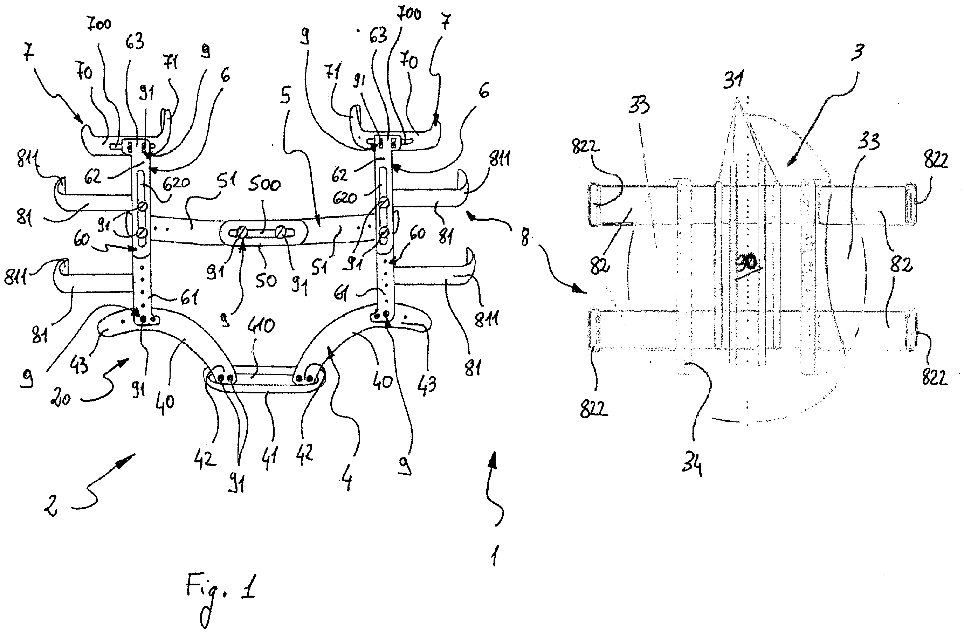

As clearly shown in Fig 1, reference numeral 1 indicates, as a whole, the orthopaedic corset of the invention.

More in particular, reference numeral 2 indicates its front portion and 3 the rear one.

Hereinafter, "front portion 2" of the corset 1 means that part intended to act on and adhere to, when worn, the thoracic-abdominal region of a patient while the "rear portion 3" is that dorsal element in contact with his/her back. In Fig. 1, said front portion 2 is schematically shown as a rigid frame 2 comprising a plurality of elements 20 with a substantially rectilinear and/or curvilinear setup made of mouldable material but sufficiently rigid and resistant to support the spine (e.g., made of metallic material such as aluminium or its alloys; of course, nothing prevents such frame from being made of carbon or its derivatives).

A plurality of "sections" connected to one another without interruption is shown of said rigid frame 2, the purposes whereof shall be clarified hereinafter with reference to a preferred embodiment of the corset 1; in particular there is shown an abdominal section 4, a thoracic section 5, a latero-abdominal section 6 and an axillary section 7, so called by virtue of the anatomical region of the torso whereon they rest and/or act.

It should be noted that in the orthopaedic corset 1 of the invention the thoracic section 5 acts at the under-pectoral region of the torso of the patient (such section being therefore called "thoracic") rather than in the sternal one (or suprasternal), thus differentiating from the prior art; this avoids (or at least reduces) that compression on the sternal region causing pain or respiratory complications to the user.

The elements 20 defining the various sections of the corset 1 are jointed to each other through coupling means already in use for the known orthopaedic corsets which also ensure those adjustments required to adapt the orthopaedic corset 1 to the physical-anatomical features of the torso of the patient. Even those aspects will be detailed during the description.

The dorsal element 3 of the corset 1 is a dorso-lumbar plate 3 having preferably a vertically extended shape and dimensions substantially equal to those of the back of the patient.

The combined action of the front portion and of the dorsal one of the corset 1 is such as to support and put the spine in hyperextension while maintaining the torso of the patient substantially straight, not subject to bending and unloaded from any stress.

What described thus far (except for what said for the thoracic plate 5) does not substantially depart from the prior art.

However, according to the invention, the dorsal element 3 and the front rigid frame 2, instead of forming a single-block body as in the current corsets on the market, are two elements normally separate but capable of being coupled and constrained to each other.

Their coupling 8 (which makes the corset 1 usable and operational) is assigned to a plurality of front straps 81 of the frame 2 and rear straps 82 of the dorso-lumbar plate 3 which are fastened at the sides of the user.

In order to better distinguish them, the front straps 81, constrained to the latero-abdominal section (6) of the frame 2, shall be called "belts 81" while the straps 82 of the dorso-lumbar plate 3 shall be called with the term of "fastening belts 82" being provided with those loops 822 on which the free ends 811 of the belts 81 are inserted and locked.

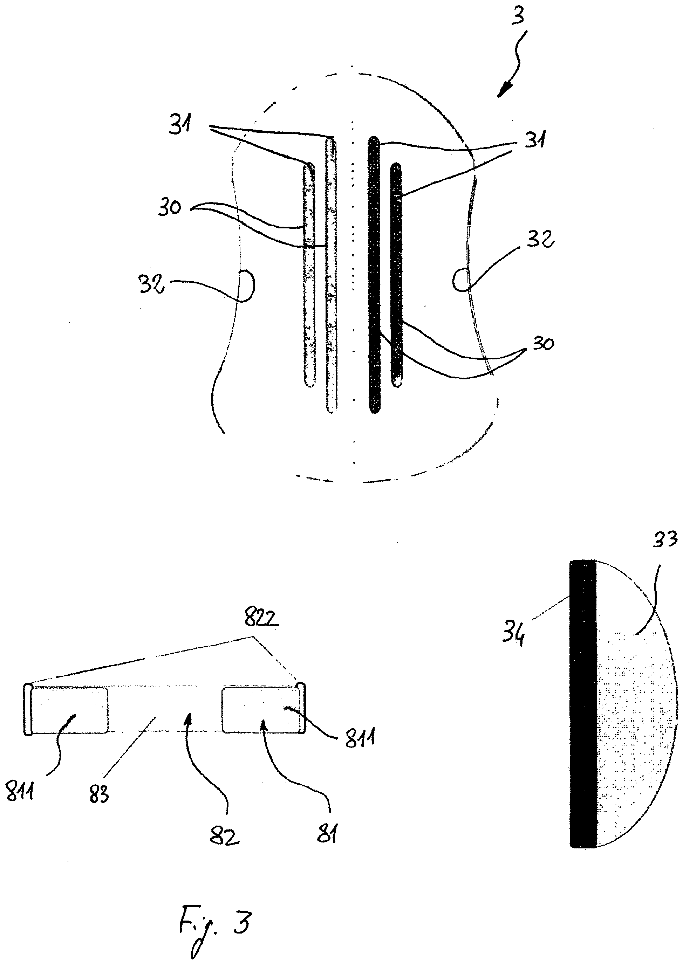

The locking of the belts 81 on the respective fastening belts 82 is ensured by known means, such as, by way of a non-limiting example, reciprocal closing means 83 of the "velcro" type (see Fig. 3).

Two pairs of front 81 and rear 82 belts are sufficient to avoid the movement and migration of the corset 1 once installed to the trunk of the user.

The application of the corset 1 of the invention to the torso of the patient affected by pathologies of the spine or subjected to surgery on the same is therefore considerably eased and may be carried out without the need for spine mobilisation; this avoids any situation of physical and emotional stress to the patient and reduces the risk of relapses or additional damages to his\her spine. Unlike the prior art, in fact, the corset 1 of the invention may be applied, without any difficulty, also to those patients forced to maintain a supine position, for example, laid in bed.

The operations for applying the corset 1 consist firstly in bring the dorso-lumbar plate 3, still "detached" from the corresponding frame 2, in contact with the back of the user, maintained in a laid position, and ensuring that the fastening belts 82 come out laterally from the same.

It has been found that for such operation it is not necessary to raise the torso of the patient in upright position, being, on the contrary, sufficient a slight rotation on his\her side of about 5-10°C; a rotation of such amount does not cause any pain or damage to the patient.

Subsequently, the front frame 2 is applied to the torso of the patient in a supine position, which is fastened by the latter to the dorso-lumbar plate 3 by means of the above-mentioned straps 81, 82.

The adjustment of the fastening of the front straps 81 on the rear ones 82 is extremely simple thanks to the large amount of reciprocal sliding points provided.

Further adjustments of the frame 2 and/or of the dorsal element 3 to the physical-anatomical features of the patient shall be possible both on the sagittal plane and on the frontal and/or transverse ones as it will be described in detail hereinafter. Now that the invention has been described in its most general aspects we shall move to describe a preferred embodiment of orthopaedic corset 1 for supporting the spine, it being understood the possibility of several changes and variants, all falling within the same inventive concept; moreover, all the components which shall be described may be replaced with technically equivalent ones, as well as the materials used, the shapes and dimensions may be any according to technical requirements.

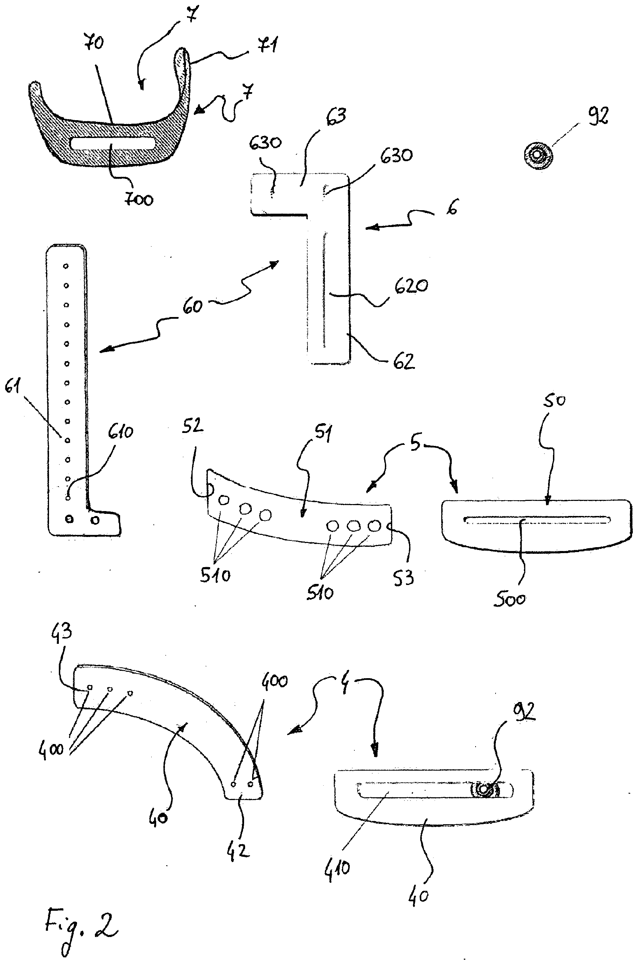

As shown in the annexed Figs. 1 and/or 2, the front abdominal section 4 previously described, comprises a pair of side iliac supports 40 which substantially follow the curvature of the iliac crests of the pelvis.

Said iliac supports 40 relieve the entire body weight, normally resting on the spine, on the pelvis of the user and, at the same time, prevent any sliding downwards of the corset 1.

The abdominal section 4 further comprises a plate 41 located at the pubic zone, therefore known as pubic plate 41.

The proximal ends 42 (relative to the sagittal plane of the patient's body) of the iliac supports 40 are constrained to the pubic plate 41 with the methods that shall be described, the distal ends 43 whereof rest, on the contrary, on the parallel vertical rods of the latero-abdominal sections 6 of the corset 1.

Without loss of generality, said latero-abdominal rods 60, located at the side of the patient and adapted to stabilize the trunk on the front plane (i.e. avoiding it to bend laterally), may consist in a single extended rod (variant not shown) or be obtained from the assembly of multiple elements, preferably the assembly of a lower bar 61 to an upper bar 62 (see details of Fig. 3).

More precisely, the lower bar 61 is constrained inferiorly to the contiguous iliac support 40 and on top to the upper bar 62, ending, in turn, with a summit appendix 63 acting as an anchoring zone to the axillary section 7 of the corset 1. As shown in Fig. 1 and/or 2, the axillary section 7 comprises a pair of under-axillary supports 70 suitably shaped in order to couple with the respective axillary cavity of the patient and ending with an "acromial thrust fork" 71 which contributes, with the rear dorso-lumbar plate 3, to put the spine in hyperextension and to release the mechanical stress acting thereon.

The above described thoracic section 5 comprises a central under-pectoral plate 50 and a pair of side thoracic sockets 51.

While the distal ends 52 (relative to the sagittal plane) of the thoracic sockets 51 are constrained to the lower bars 61 of the rods 60, the proximal ones 53 rest on said under-pectoral plate 50.

For the reasons that shall be understood just below, one or more openings, in the form of through holes 610, 510, 400 or slots 620, 630, 500, 410, 700 are provided at the terminal ends of each of the above elements of the frame 2, through which the hooking, through known coupling means 9, is obtained with the contiguous element.

As well known to the man skilled in the art, said coupling means 9 may consist in a screw-nut screw coupling, preferably of the "screw 91 - bushing 92" type, being such system the one that best fits the connection of reduced thickness components (of about 1-2 mm) as in the case of the frame 2 of the invention. Said coupling is obtained by means of a screw 91 the threaded stem whereof is inserted in the "passage" (not explicitly illustrated in the annexed figures) defined by the superimposition of two through holes 610, 510, 400 (and/or a hole and a slot and/or two slots) of the elements to be connected and fastened on a bushing 92 located at the exit of said passage.

More precisely, a plurality of aligned holes 610 is provided along the lower bar 61 of the latero-abdominal rods 60, whereon, for the above purposes, the holes 400, 510 obtained respectively on at least the distal ends of the iliac supports 40 and of the thoracic sockets 51 or those (not shown) of the straps 81 are interfaced and made cooperating.

Each hole 610, 510, 400 determines, therefore, a possible reciprocal fixing point for said elements, by defining a wide range of sizes for the corsets 1 adjustable according to the physical features of the user.

When assembling the frame 2, the slots 620 and 630 of said upper bar 62 are made cooperating respectively with at least a hole 610 of the lower bar 61 and with the slot 700 of the axillary support 70.

Likewise, the slots 410 and 500 provided on the pubic 41 and thoracic 50 plates interface to the respective pierced proximal ends of the iliac supports 40 and of the thoracic sockets 51.

The above described slotted elements may be made to slide on those to which they are connected (prior to the obvious "loosening" of the respective "screw-bushing" coupling) allowing a precise and quick adjustment of the frame 2 according to the physical-anatomical features of the trunk of the patient.

For example, by making the slotted upper bars 62 of the latero-abdominal rods 60 to slide on the respective lower bars 61 or the pubic 41 and/or thoracic 50 plate on the respective iliac supports 40 and thoracic sockets 51 it is possible to adjust respectively the height and width of the frame 2.

Finally, the slot 700 of the axillary support 70, by means of which the connection to the underlying latero-abdominal rod 60 is obtained, instead allows a quick adjustment of its width relative to the axillary cavity.

Of course, nothing prevents the possibility of providing simplified coupling means, for example of the type comprising threaded screws that can be fastened on dedicated threaded holes of the elements of the frame 2 to be assembled (or similar/equivalent) or through buttons, but at the expense of its possible adjustments to the user body.

Let's now describe in detail the second portion of the orthopaedic corset 1, capable of being constrained to the frame 2, which, as already mentioned, consists in a dorso-lumbar plate 3 acting as a rear support and hyperextensor for the trunk of the patient avoiding its forward bending and reducing the risk of Said dorso-lumbar plate 3 is preferably a pad comprising pockets 30 adapted to accommodate a plurality of paravertebral stiffening slats 31 (removable/insertable in said pockets 30 depending on the stiffness degree required for the dorso-lumbar element 3 and/or in case of maintenance or replacement).

Those fastening straps 82, whereon the straps 81 of the frame 2 fasten, are also applied at the opposite lateral sides 32 of said dorso-lumbar plate 3.

Velcro fastening means allows fixing said straps 82 at various heights, depending on the position taken by the front straps 81 of the latero-abdominal rods 60 of the frame 2.

The dorsal surface of said dorso-lumbar plate 3 may be increased if needed by means of any padded appendices 33, applicable at its said lateral sides 32 through velcro 34 or other equivalent means (e.g. buttons); in that case, said appendices 33 may lead the above straps 82 to ensure their height adjustment. Finally, it is useful to clarify that said frame 2 and said dorso-lumbar plate 3 may be, at least in part, padded (e.g. at the acromial thrust fork and/or dorso-lumbar plate) or coated with fabric material that is washable, hygienic and of high aesthetic value

In conclusion, it has been thus seen that the above described bivalve corset 1 achieves the previously noted objects, in particular the possibility of application without the need for moving the patient affected by pathologies of the spine.

In other words, it is possible to apply the corset 1 to patients who have just undergone surgery or affected by extremely debilitating traumas/pathologies keeping them in a supine position.

As a consequence, with the corset 1 of the invention the risks of relapse and/or increase of pain which may arise from raising at least the torso of the patient are eliminated.

The high adjustability and adaptability of the frame 2 and of the relative dorsal plate 3 to the physical-anatomical features of the patient ensure a high comfort of use to the corset 1.

It is finally noted that the presence of easily removable elements, without the need for using specific tools, makes the corset 1 of the invention easy to repair, reconfigure and/or wash (e.g. for its padding and/or textile upholstery).

## Claims

1. Orthopaedic corset (1) for the hyperextension of the spine of a user suffering from diseases or subjected to trauma and/or surgery, of the type comprising:
- a front portion intended to act on the thoracic-abdominal region of the torso of said user, said front portion consisting in a front frame (2) comprising a plurality of elements (20) constrained and connected to form at least one abdominal section (4), a thoracic section (5), a latero-abdominal section (6) and an axillary section (7),
- a rear portion capable of being placed in contact with the back of said user, said rear portion being a dorso-lumbar element (3) said front frame (2) and said dorso-lumbar element (3) being components that are normally separated from each other but capable of being mutually connected and constrained to make said corset (1) operating, said connection being realised and localised, by means of special coupling means (8), at each side of said user
**characterised in that**
said thoracic section (5) being located and acting, in use, at the under-pectoral region of the torso of said user.

2. Orthopaedic corset (1) according to the previous claim,
**characterised in that**
said thoracic section (5) further comprises a central thoracic plate (50) and a pair of side thoracic sockets (51) having proximal and distal ends, the distal ends (52) of said thoracic sockets (51) being connected to said latero-abdominal section (6) of said frame (2), the proximal ends (53) to said central thoracic plate (50).

3. Orthopaedic corset (1) according to any previous claim,
**characterised in that**
- said abdominal section (4) comprises a pair of opposed side iliac supports (40) having proximal and distal ends, and a central pubic plate (41), the distal ends (43) of said iliac supports (40) being connected to said latero-abdominal section (6) of said frame (2), the proximal ends (42) to said pubic plate (41),
- said axillary section (7) comprises a pair of axillary supports (70), said axillary supports (70) being connected to a respective summit appendix of said latero-abdominal section (6) and comprising an acromial thrust fork (71),
- said latero-abdominal section (6) consists in a pair of vertical rods (60), parallel to each other, each acting in the proximity of a side of said user, when the corset is in use.

4. Orthopaedic corset (1) according to the previous claim,
**characterised in that**
each vertical rod (60) of said latero-abdominal section consists in a single rod.

5. Orthopaedic corset (1) according to claim 3,
**characterised in that**
each vertical rod (60) of said latero-abdominal section includes a lower bar (61) and an upper bar (62), said lower bar (61) being connected inferiorly to the corresponding said iliac support (40) and at the top to said upper bar (62), the latter in turn connected to said axillary support (70).

6. Orthopaedic corset (1) according to at least claims 2 to 5,
**characterised in that**
said connections are made via screw-nut screw coupling (9) of the screw (91) - bush (92) type.

7. Orthopaedic corset (1) according to any previous claim,
**characterised in that**
said rear dorso-lumbar element (3) is a dorso-lumbar plate (3) comprising a plurality of pockets (30) adapted to accommodate paravertebral stiffening slats (31), said slats (31) being removable/insertable from/in said pockets (30) depending on the stiffness degree required for the said dorso-lumbar plate (3) and/or in case of maintenance or replacement.

8. Orthopaedic corset (1) according to the previous claim, **characterised in that** the corset further comprises side appendices (33), said side appendices (33) can be constrained to said dorso-lumbar element (3) to increase the dorsal surface thereof.

9. Orthopaedic corset (1) according to one or more of the previous claims,
**characterised in that**
said coupling means (8) of said front frame (2) and of said rear dorso-lumbar element (3) of said corset (1) comprise one or more front straps (81) constrained to said latero-abdominal section (6) of said front frame (2) and a corresponding number of rear straps (82) constrained to said dorso-lumbar element (3),
each of said one or more rear straps (82) comprising a loop (822) on which the free ends (811) of said one or more front straps (81) are inserted and locked,
said front (81) and rear straps (82) being able to be adjusted and moved in height along said latero-abdominal section (6) and said dorso-lumbar element (3).

10. Orthopaedic corset (1) according to any previous claim,
**characterised in that** at least at the terminal ends of each of said elements (20) of said front frame (2) there are provided one or more openings in the shape of through holes (610; 510; 400) or slots (620; 630; 500; 410; 700) through which the coupling is realised through coupling means (9; 91; 92) between one of said elements (20) and the contiguous one, said coupling means (9; 91, 92) consisting in a screw-nut screw coupling, preferably of the "screw (92) - bushing (92) type.

## Patentansprüche

1. Orthopädisches Korsett (1) für die Hyperextension der Wirbelsäule eines Benutzers, der an Krankheiten leidet oder ein Trauma erlitten hat und/oder einer Operation unterzogen wurde, des Typs, umfassend:
- einen vorderen Teil (2), der dazu bestimmt ist, auf den Thorax-Abdominalbereich des Rumpfes des Benutzers einzuwirken, wobei der vordere Teil (2) aus einem Vorderrahmen (2) besteht, der mehrere Elemente (20) aufweist, die befestigt und verbunden sind, um mindestens einen Abdominalabschnitt (4), einen Thoraxabschnitt (5), einen lateralen Abdominalabschnitt (6) und einen Axillarabschnitt (7) zu bilden,
- einen hinteren Teil (3), der geeignet ist, mit mit dem Rücken des Benutzers in Kontakt gebracht zu werden, wobei der hintere Teil (3) ein dorsolumbales Element (3) ist,
wobei der Vorderrahmen (2) und das dorsolumbale Element (3) Komponenten sind, die normalerweise voneinander getrennt sind, aber geeignet sind, miteinander verbunden und befestigt zu werden, damit das Korsett (1) funktioniert, wobei die Verbindung durch spezielle Verbindungsmittel (8) auf jeder Seite des Benutzers erstellt und angeordnet ist,
**dadurch gekennzeichnet, dass**
der Thoraxabschnitt (5) im Einsatz an dem Unterbrustbereich des Rumpfes des Benutzers angeordnet ist und wirkt.

2. Orthopädisches Korsett (1) nach dem vorhergehenden Anspruch,
**dadurch gekennzeichnet, dass**
der Thoraxabschnitt (5) ferner eine zentrale Thoraxplatte (50) und ein Paar seitliche thorakale Aufnahmen (51) aufweist, die proximale und distale Enden aufweisen, wobei die distalen Enden (52) der thorakalen Aufnahmen (51) mit dem lateralen Abdominalabschnitt (6) des Rahmens (2), die proximalen Enden (53) mit der zentralen Thoraxplatte (50) verbunden sind.

3. Orthopädisches Korsett (1) nach einem der vorhergehenden Ansprüche,
**dadurch gekennzeichnet, dass**
- der Abdominalabschnitt (4) ein Paar gegenüberliegende seitliche Iliakalstützen (40), die proximale und distale Enden aufweisen, und eine zentrale Schambeinplatte (41) aufweist, wobei die distalen Enden (43) der Iliakalstützen (40) mit dem lateralen Abdominalabschnitt (6) des Rahmens (2), die proximalen Enden (42) mit der Schambeinplatte (41) verbunden sind,
- der Axillarabschnitt (7) ein Paar Axillarstützen (70) aufweist, wobei die Axillarstützen (70) mit einem jeweiligen oberen Anhang des lateralen Abdominalabschnitts (6) verbunden sind und eine Akromial-Schubgabel (71) aufweisen,
- wobei der laterale Abdominalabschnitt (6) aus einem Paar vertikaler Stangen (60), die parallel zueinander sind, besteht, wobei jede in der Nähe von einer Seite des Benutzers wirkt, wenn das Korsett benutzt wird.

4. Orthopädisches Korsett (1) nach dem vorhergehenden Anspruch,
**dadurch gekennzeichnet, dass**
jede vertikale Stange (60) des lateralen Abdominalabschnitts aus einer einzigen Stange besteht.

5. Orthopädisches Korsett (1) nach Anspruch 3,
**dadurch gekennzeichnet, dass**
jede vertikale Stange (60) des lateralen Abdominalabschnitts eine untere Leiste (61) und eine obere Leiste (62) aufweist, wobei die untere Leiste (61) unten mit der entsprechenden Iliakalstütze (40) und oben mit der oberen Leiste (62) verbunden ist, wobei die Letztere ihrerseits mit der Axillarstütze (70) verbunden ist.

6. Orthopädisches Korsett (1) nach einem der Ansprüche 2 bis 5,
**dadurch gekennzeichnet, dass**
die Verbindungen über eine Schraube-Mutter-Schraubverbindung (9) vom Typ der Schraube (91)-Buchse (92)-Verbindung erstellt sind.

7. Orthopädisches Korsett (1) nach einem der vorhergehenden Ansprüche,
**dadurch gekennzeichnet, dass**
das hintere dorsolumbale Element (3) eine dorsolumbale Platte (3) ist, die mehrere Taschen (30) aufweist, die geeignet sind, paravertebrale Versteifungsstäbe (31) aufzunehmen, wobei die Stäbe (31) je nach dem Grad der Steifigkeit, der für die dorsolumbale Platte (3) erforderlich ist, und/oder im Fall der Wartung oder des Austauschs von den Taschen (30) abnehmbar/darin einfügbar sind.

8. Orthopädisches Korsett (1) nach dem vorhergehenden Anspruch,
**dadurch gekennzeichnet, dass** das Korsett ferner seitliche Anhänge (33) aufweist, wobei die Anhänge (33) an dem dorsolumbale Element (3) befestigt werden können, um die Dorsalfläche davon zu vergrößern.

9. Orthopädisches Korsett (1) nach einem oder mehreren der vorhergehenden Ansprüche,
**dadurch gekennzeichnet, dass**
die Verbindungsmittel (8) des Vorderrahmens (2) und das hintere dorsolumbale Element (3) des Korsetts (1) einen oder mehrere vordere Riemen (81), die an dem lateralen Abdominalabschnitt (6) des Vorderrahmens (2) befestigt sind, und eine entsprechende Anzahl an hinteren Riemen (82) aufweisen, die an dem dorsolumbalen Element (3) befestigt sind,
wobei jeder des einen oder der mehreren hinteren Riemen (82) eine Schlaufe (822) aufweist, in die die freien Enden (811) des einen oder der mehreren vorderen Riemen (81) eingefügt und geschlossen sind, wobei die vorderen (81) und hinteren Riemen (82) geeignet sind, eingestellt und in der Höhe entlang des lateralen Abdominalabschnitts (6) und des dorsolumbalen Elements (3) bewegt zu werden.

10. Orthopädisches Korsett (1) nach einem der vorhergehenden Ansprüche,
**dadurch gekennzeichnet, dass** mindestens an den terminalen Enden der Elemente (20) des Vorderrahmens (2) eine oder mehrere Öffnungen in der Form von Durchgangslöchern (610; 510; 400) oder Schlitzen (620; 630; 500; 410; 700) vorgesehen sind, durch die die Verbindung mit Verbindungsmitteln (9; 91; 92) zwischen einem der Elemente (20) und dem angrenzenden erstellt wird, wobei die Verbindungsmittel (9; 91; 92) in einer Schraube-Mutter-Schraubverbindung, vorzugsweise vom Typ der Schraube (92)-Buchse (92)-Verbindung, bestehen.

## Revendications

1. Corset orthopédique (1) pour l'hyperextension de la colonne vertébrale d'un utilisateur souffrant de maladies ou soumis à un traumatisme et/ou à une intervention chirurgicale, du type comprenant :
- une partie avant (2) destinée à agir sur la région thoraco-abdominale du torse dudit utilisateur, ladite partie avant (2) consistant en un cadre avant (2) comprenant une pluralité d'éléments (20) liés pour former au moins une section abdominale (4), une section thoracique (5), une section latéro-abdominale (6) et une section axillaire (7),
- une partie arrière (3) apte à être mise en contact avec le dos dudit utilisateur, ladite partie arrière (3) étant un élément dorso-lombaire (3) ledit cadre avant (2) et ledit élément dorso-lombaire (3) étant des composants normalement séparés l'un de l'autre mais pouvant être reliés entre eux et contraints à faire fonctionner ledit corset (1), ladite connexion étant réalisée de moyens de couplage spéciaux (8), de chaque côté dudit utilisateur
**caractérisé en ce que**
ladite section thoracique (5) étant située et agissant, en cours d'utilisation, dans la région sous-pectorale du torse dudit utilisateur.

2. Corset orthopédique (1) selon la revendication précédente,
**caractérisé en ce que**
ladite section thoracique (5) comprend en outre une plaque thoracique centrale (50) et une paire de cavités thoraciques latérales (51) ayant des extrémités proximale et distale, les extrémités distales (52) desdites cavités thoraciques (51) étant reliées auxdites section abdominale (6) dudit cadre (2), les extrémités proximales (53) à ladite plaque thoracique centrale (50).

3. Corset orthopédique (1) selon l'une quelconque des revendications précédentes,
**caractérisé en ce que**
- ladite section abdominale (4) comprend une paire de supports iliaques latéraux opposés (40) ayant des extrémités proximale et distale et une plaque pubienne centrale (41), les extrémités distales (43) desdits supports iliaques (40) étant reliées à ladite section latéro-abdominale (6) dudit cadre (2), les extrémités proximales (42) à ladite plaque pubienne (41),
- ladite section axillaire (7) comprend une paire de supports axillaires (70), lesdits supports axillaires (70) étant reliés à un appendice de sommet respectif de ladite section latéro-abdominale (6) et comprenant une fourche de poussée acromiale (71),
- ladite section latéro-abdominale (6) consiste en une paire de tiges verticales (60), parallèles l'une à l'autre, agissant chacune à proximité d'un côté dudit utilisateur, lorsque le corset est utilisé.

4. Corset orthopédique (1) selon la revendication précédente,
**caractérisé en ce que**
chaque tige verticale (60) de ladite section latéro-abdominale consiste en une seule tige.

5. Corset orthopédique (1) selon la revendication 3,
**caractérisé en ce que**
chaque tige verticale (60) de ladite section latéro-abdominale comprend une barre inférieure (61) et une barre supérieure (62), ladite barre inférieure (61) étant reliée en dessous dudit support iliaque (40) correspondant et en haut ladite barre supérieure (62), celle-ci à son tour reliée audit support axillaire (70).

6. Corset orthopédique (1) selon au moins les revendications 2 à 5,
**caractérisé en ce que**
lesdites connexions se font par vissage à vis (9) du type vis (91) - douille (92).

7. Corset orthopédique (1) selon l'une quelconque des revendications précédentes,
**caractérisé en ce que**
ledit élément dorso-lombaire arrière (3) est une plaque dorso-lombaire (3) comprenant une pluralité de poches (30) adaptées pour recevoir des lattes de rigidification paravertébrales (31), lesdites lattes (31) pouvant être retirées/insérées depuis/dans lesdites poches (30) en fonction du degré de rigidité requis pour ladite plaque dorso-lombaire (3) et/ou en cas de maintenance ou de remplacement.

8. Corset orthopédique (1) selon la revendication précédente,
**caractérisé en ce que** le corset comprend en outre des appendices latéraux (33), lesdits appendices latéraux (33) pouvant être limités audit élément dorso-lombaire (3) pour en augmenter la surface dorsale.

9. Corset orthopédique (1) selon une ou plusieurs des revendications précédentes,
**caractérisé en ce que**
lesdits moyens de couplage (8) dudit cadre avant (2) et dudit élément dorso-lombaire arrière (3) dudit corset (1) comprennent une ou plusieurs sangles avant (81) contraintes à ladite section latéro-abdominale (6) de ledit cadre avant (2) et un nombre correspondant de sangles arrière (82) étant contraint audit élément dorso-lombaire (3), chacune desdites une ou plusieurs sangles arrière (82) comprenant une boucle (822) sur laquelle les extrémités libres (811) de ladite ou desdites sangles avant (81) sont insérées et verrouillées,
lesdites sangles avant (81) et arrière (82) pouvant être ajustées et déplacées en hauteur le long de ladite section latéro-abdominale (6) et dudit élément dorso-lombaire (3).

10. Corset orthopédique (1) selon l'une quelconque des revendications précédentes,
**caractérisé en ce qu** 'au moins aux extrémités terminales de chacun desdits éléments (20) dudit cadre avant (2) sont prévues une ou plusieurs ouvertures en forme de trous traversants (610 ; 510 ; 400) ou de fentes (620 ; 630 ; 500 ; 410 ; 700) à travers lequel l'accouplement est réalisé par des moyens de couplage (9 ; 91 ; 92) entre l'un desdits éléments (20) et le joint, lesdits moyens de couplage (9; 91, 92) consistant en une vis à visser en noyer, de préférence du type à vis (92) - douille (92).
